Europäisches Patentamt

(19) European Patent Office

Office européen des brevets

(11) Veröffentlichungsnummer: **0 226 822**
**A2**

## (12) EUROPÄISCHE PATENTANMELDUNG

(21) Anmeldenummer: 86116023.2

(22) Anmeldetag: 18.11.86

(51) Int. Cl.⁴: **G 01 N 21/59**
**G 01 N 33/00**

(30) Priorität: 21.11.85 DE 3541165

(43) Veröffentlichungstag der Anmeldung:
01.07.87 Patentblatt 87/27

(84) Benannte Vertragsstaaten:
AT CH DE FR GB LI

(71) Anmelder: Hellige GmbH
Postfach 728 Heinrich-von-Stephan-Strasse 4
D-7800 Freiburg im Breisgau(DE)

(72) Erfinder: Schmidtke, Gerhard, Dr. Dipl.-Phys.
Gilgenmatten 35
D-7800 Freiburg(DE)

(72) Erfinder: Preier, Horst, Prof. Dipl.-Phys.
Keltenring 100
D-7815 Kirchzarten(DE)

(72) Erfinder: Ullrich, Georg J, Dipl.-Phys.
Mettackerweg 84
D-7800 Freiburg(DE)

(74) Vertreter: Patentanwälte TER MEER - MÜLLER -
STEINMEISTER
Mauerkircherstrasse 45
D-8000 München 80(DE)

(54) Vorrichtung zur kontinuierlichen Bestimmung von Konzentrationsänderungen in Stoffgemischen.

(57) Bei einer Vorrichtung zur kontinuierlichen transkutanen Überwachung des Glukosespiegels im Blut eines Patienten wird dessen Ohrläppchen mit Licht durchstrahlt, wobei die glukosespezifische Absorption mit Hilfe einer Detektoranordnung erfaßt wird. Die verwendete Strahlungsquelle (1) ist eine breitbandige Lampe, deren Licht über eine Optik in drei Lichtkanäle mit jeweils einem eine individuelle Frequenz aufweisenden Chopper (2, 3, 4) einem Filter (8, 9, 10) und einem Lichtwellenleiter (12, 13, 14) in einen Ohrclip (15) eingekoppelt ist. Das Ausgangssignal der Detektoranordnung (16) wird über auf die Chopperfrequenzen abgestimmte Lock-in-Verstärker (18, 19, 20, 22) an eine Auswerteschaltung (31) angeschlossen. Die Transmissionswellenlänge des ersten Filters (8) liegt in einem hauptsächlich auf die Anzahl der roten Blutkörperchen ansprechenden Bereich, die des zweiten Filters (9) in einem Bereich, wo Glukose nur schwach absorbiert, und die des dritten Filters (10) im Bereich einer Absorptionsbande von Glukose. Auf diese Weise können Schwankungen in der Schichtdicke des Ohrläppchens sowie der Helligkeit der Strahlungsquelle (1) erfaßt werden und dadurch bedingte Auswertefehler vermieden werden.

./...

Fig.1

# Vorrichtung zur kontinuierlichen Bestimmung von Konzentrationsänderungen in Stoffgemischen

Die Erfindung betrifft eine Vorrichtung zur kontinuierlichen Bestimmung von Konzentrationsänderungen in Stoffgemischen, mit einer Strahlungsquelle, deren Licht die Meßprobe durchstrahlt und von einer Detektoranordnung zur Bestimmung der stoffspezifischen Absorptionsmessung erfaßt wird.

Eine derartige Vorrichtung zur Bestimmung der Glukosekonzentration im Blut eines Patienten ist aus der DE-OS 27 24 543 bekannt und verfügt über eine Strahlungsquelle, die Licht nur einer Wellenlänge aussendet. Nach Durchgang durch die als Ohrclip ausgebildete Meßprobe durchquert das Licht einen Filter, um mögliche Störeinflüsse von Streulicht auszuschalten. Die Detektoranordnung setzt das Lichtsignal in ein elektrisches Signal um, das nach Verstärkung einem Anzeigegerät zugeführt wird. Wenn in einer solchen Anordnung die Absorption von Glukose in-vivo erfaßt werden soll, zeigt sich, daß die für die Praxis erforderlichen Langzeitstabilitäten und Genauigkeiten nicht realisierbar sind. Insbesondere ergeben sich störende Intensitätsschwankungen dadurch, daß die Dicke des im Ohrclip befindlichen Ohrläppchens eines Patienten sich entsprechend dem Pulsschlag periodisch verändert, wodurch periodisch eine Glukosekonzentrationsänderung vorgetäuscht wird. Andere Instabilitäten ergeben sich dadurch, daß sich die Lichtquelle bei Betriebsdauern von mehreren Stunden in ihrer Strahlungsintensität verändert.

In der US-PS 4 086 915 ist eine Vorrichtung zum Durchstrahlen des Ohrläppchens eines Patienten beschrieben, bei der mehrere verschiedene Lichtquellen verwendet

werden, die über eine Muliplexer- und Demultiplexeranordnuug mit einer Auswerteeinheit verbunden sind um Störfaktoren auszuschalten. Ein anderes Ohr-Oximeter ist aus der DE-AS 27 41 981 bekannt, bei dem der Einfluß von Störfaktoren durch Verwendung von drei Meßfrequenzen weitgehend ausgeschaltet wird. Auch hierbei werden jedoch mehrere verschiedene Lichtquellen verwendet.

Ausgehend von diesem Stand der Technik liegt der Erfindung die Aufgabe zugrunde, eine Vorrichtung zur kontinuierlichen Bestimmung von Konzentrationsänderungen in Stoffgemischen, insbesondere zur Überwachung des Glukosespiegels im Blut eines Patienten zu schaffen, die über längere Zeit eine kontinuierliche Bestimmung geringer Konzentrationsänderungen trotz der sich ändernden Schichtdicke des Stoffgemisches bzw. des Blutes im Ohr gestattet.

Diese Aufgabe wird erfindungsgemäß dadurch gelöst, daß die Strahlungsquelle eine breitbandige Lampe ist, deren Licht über eine Optik in mehrere Lichtkanäle mit jeweils einem eine individuelle Frequenz aufweisenden Chopper, einem eine vorbestimmte Transmissionswellenlänge auszeichnenden Filter und einem Lichtwellenleiter in die Meßprobe eingekoppelt ist, daß das Ausgangssignal der Detektoranordnung über den Lichtkanälen zugeordnete auf die Chopperfrequenzen abgestimmte Lock-in-Verstärker an eine Auswertevorrichtung angeschlossen ist und daß die Transmissionswellenlänge des ersten Filters in einem hauptsächlich auf die Probenschichtdicke ansprechenden Bereich, die des zweiten Filters in einem Bereich, wo die Meßprobe nur schwach absorbiert, und die der übrigen Filter im Bereich der Absorptionsbanden der Stoffe liegt, deren Konzentrationsänderungen bestimmt werden sollen.

Die Detektoranordnung weist wenigstens einen von der Strahlung der Lichtkanäle beleuchteten Detektor auf, dessen Ausgangssignal den einzelnen Lichtkanälen zugeordnete, mit den Choppern synchronisierte Lock-in-Verstärker speist, deren den einzelnen Lichtkanälen zugeordnete Ausgangssignale eine Auswerteschaltung beaufschlagen.

Eine noch höhere Empfindlichkeit unter Ausnutzung der durch z.B. Glukose verursachten Brechung wird dann erreicht, wenn die Detektoranordnung eine Vielzahl von Detektorelementen aufweist, die rotationssymmetrisch zur optischen Achse des die Meßprobe verlassenden Lichtbündels als scheibenförmiges Zentralelement mit konzentrischen Ringelementen geschaltet sind, wobei dem Zentralelement sowie jedem Ringelement eine Auswerteeinheit mit für jeden Lichtkanal ausgelegten Lock-in-Verstärkern zugeordnet sind.

Die Auswertung dreier von einer einzigen Strahlungsquelle aufgehenden Lichtkanäle gestattet neben der Erfassung einer Stoffkonzentration eine Kompensation der von unterschiedlichen Schichtdicken beeinflußten Intensitätsänderungen sowie der durch Instabilitäten der Lichtquelle bedingten Intensitätsänderungen. Auf diese Weise können sehr geringe Konzentrationsänderungen zuverlässig erfaßt werden, wobei auch eine sich über Stunden hinziehende Konzentrationsbestimmung bzw. eine in-vivo-Überwachung der Glukosekonzentration im Ohrläppchen eines Patienten möglich ist.

Zweckmäßige Weiterbildungen und Ausgestaltungen der Erfindung sind in den Unteransprüchen gekennzeichnet.

Nachfolgend wird die Erfindung anhand von in der Zeichnung dargestellten Ausführungsbeispielen näher erörtert. Es zeigen:

Fig. 1    ein Ausführungsbeispiel der Erfindung mit drei Lichtkanälen und einem einzigen Detektor,

Fig. 2    ein Ausführungsbeispiel der Erfindung mit drei Lichtkanälen und mit einer Vielzahl von konzentrisch angeordneten ringförmigen Detektorelementen zur Erfassung des die Meßprobe verlassenden Lichtes,

Fig. 3    ein Ausführungsbeispiel der Erfindung mit vier Lichtkanälen und einem einzigen Detektor und

Fig. 4    ein Ausführungsbeispiel der Erfindung mit vier Lichtkanälen und einem die Lichtverteilung erfassenden Detektor aus mehreren konzentrisch angeordneten ringförmigen Detektorelementen.

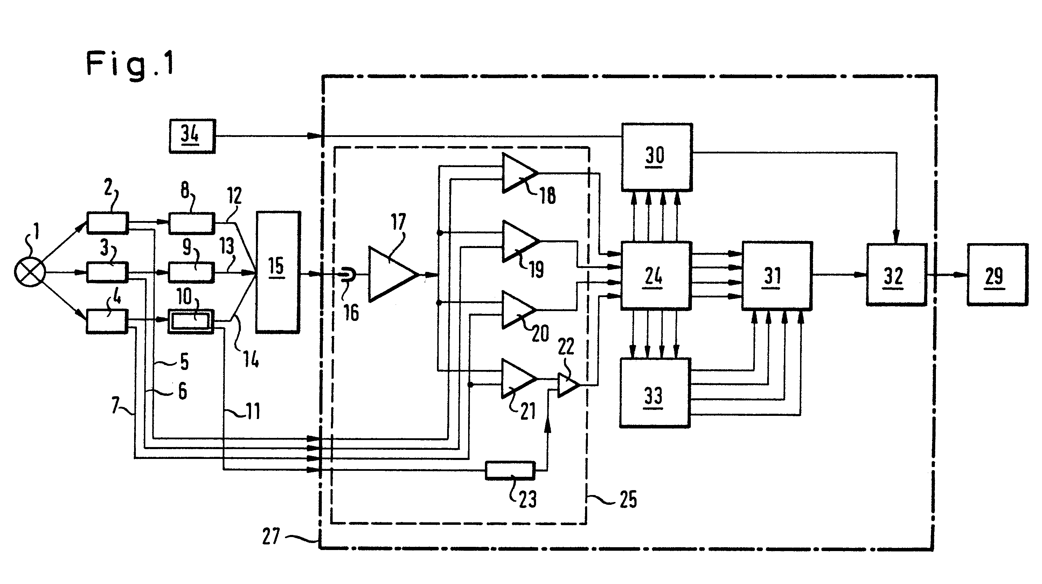

Die in Fig. 1 dargestellte Vorrichtung zur kontinuierlichen Bestimmung von Konzentrationsänderungen bzw. zur transkutanen in-vivo-Überwachung des Glukosespiegels im Blut eines Patienten verfügt über eine Strahlungsquelle 1, die beispielsweise eine Halogenlampe sein kann. Das Licht der Strahlungsquelle 1 gelangt über eine in der Zeichnung nicht dargestellte Optik mit drei Linsen zu einem Chopperrad mit drei verschiedenen Segmentierungen, die einen ersten Chopper 2, einen zweiten Chopper 3 und dritten Chopper 4 bilden. Das aus den Linsen austretende breitbandige Licht der Strahlungsquelle 1 wird von den Choppern 2 bis 4 regelmäßig und überlappend unterbrochen. Die geometrische Anordnung kann so gewählt sein, daß die drei das Chopperrad beleuchtenden Linsen im Dreieck angeordnet sind. Die Segmentierung des Chopperrades ist für jedes der in Fig. 1 eingezeichneten und von der breitbandigen Strahlungsquelle 1 ausgehenden Lichtbündel verschieden, z.B. siebzehnfach, elffach und einfach, wobei eine siebzehnfache Segmentierung bedeutet, daß siebzehn Öffnungen und siebzehn Abdeckungen im Chopperrad vorgesehen sind. Um eine Reduzierung von interferierbaren Oberwellen zu erreichen, wird für die Segmentierung des Chopperrades ein Primzahlenverhältnis bevorzugt. Den die Chopper 2, 3 und 4 bildenden Segmentierungen auf dem Chopperrad sind Lichtschranken zugeordnet, durch die Steuersignale erzeugt werden, deren Frequenzen jeweils den Chopperfrequenzen der Chopper 2, 3 und 4 zugeordnet sind. Über Leitungen 5, 6, 7 werden die Steuersignale der Chopper 2, 3, 4 ausgekoppelt.

Die Chopper 2, 3, 4 bilden die ersten Elemente dreier Lichtkanäle mit unterschiedlichen Wellenlängen und unterschiedlichen durch die Chopper 2, 3, 4 festgelegten Modulationsfrequenzen.

Das durch den Chopper 2 in den ersten Lichtkanal eingespeiste Licht gelangt zu einem Interferenzfilter 8, dessen Transmissionswellenlänge schichtdickenspezifisch ist, bzw. bei 805 nm liegt. Licht dieser Wellenlänge wird beim Durchqueren von Blut in Abhängigkeit von der Anzahl der roten Blutkörperchen im Lichtweg unabhängig von der Glukosekonzentration geschwächt. Aus diesem Grunde dient Licht der Wellenlänge von 805 nm zur Erfassung von Veränderungen der effektiven Schichtdicke des Blutes.

Über den Chopper 3 gelangt ein Teil des Lichtes der breitbandigen Strahlungsquelle 1 zu einem zweiten Interferenzfilter 9, dessen Transmissionswellenlänge im Bereich niedriger Absorption der Meßprobe bzw. um 1300 nm liegt. In diesem Bereich absorbiert Glukose nur schwach, weshalb das Licht im zweiten Lichtkanal verwendet werden kann, um Veränderungen von Gerätepara-

metern, wie die der Lichtintensität, zu erfassen.

Das dem dritten Chopper 4 zugeordnete dritte Interferenzfilter 10 hat eine Transmissionswellenlänge im Bereich einer Absorptionsbande des zu überwachenden Stoffes, z.B. von 1600 nm. Dieser Bereich überdeckt eine Absorptionsbande von Glukose.

Das dritte Interferenzfilter 10 kann so ausgebildet sein, daß die Transmissionswellenlänge periodisch mit einer vorgegebenen Frequenz von beispielsweise 1 kHz verändert wird. Eine solche Wellenlängenmodulation, die auf das 1,1-fache der Halbwertsbreite der Absorptionsbande abgestimmt ist, gestattet die Realisierung der aus der Derivativ-Spektroskopie bekannten Verfahren. Die Modulationsfrequenz von 1 kHz muß erheblich größer sein als die oben erwähnten Chopperfrequenzen. Durch den Einsatz der Derivativ-Spektroskopie ist es möglich, im dritten Lichtkanal eine besonders empfindliche und stabile Konzentrationsmessung insbesondere von Glukose durchzuführen. Über eine Leitung 11 wird ein der Modulationsfrequenz entsprechendes Signal ausgekoppelt.

An den Ausgängen der Interferenzfilter 8, 9 und 10 stehen mit unterschiedlichen Frequenzen gechoppte Strahlungsquellen unterschiedlicher Wellenlängen zur Verfügung. Das Licht der drei Lichtkanäle wird hinter den Interferenzfiltern 8, 9, 10 in Lichtwellenleiter 12, 13, 14 eingespeist und mit deren Hilfe zu einer Probenhalterung 15 geführt, die in an sich bekannter Weise insbesondere als Ohrclip ausgebildet ist, das auf das Ohrläppchen eines Patienten aufgeklemmt ist. Die Ausgangsenden der Lichtwellenleiter 12, 13, 14 sind in der Probenhalterung 15 auf das Zentrum der Meßprobe, insbesondere des Ohrläppchens gerichtet. Das aus der Meßprobe austretende Licht, welches Licht aus allen drei Lichtkanälen enthält, gelangt bei dem in Fig. 1 dargestellten Ausführungsbeispiel zu einem einzelnen Detektor 16, dessen Ausgangssignal über einen Verstärker 17 den drei Lichtkanälen zugeordnete Lock-in-Verstärker 18, 19, 20 speist. Über die Leitungen 5, 6, 7 gelangen die von den Choppern 2, 3, 4 erzeugten Taktsignale zu den Lock-in-Verstärkern 18, 19, 20 und gestatten auf diese Weise eine Separierung der den drei Lichtkanälen zugeordneten Signale.

Der Ausgang des Verstärkers 17 ist weiterhin mit dem Eingang eines Boxcar-Verstärkers 21 (Verstärker mit Torschaltung) verbunden, dessen Ausgangssignal einen weiteren Lock-in-Verstärker 22 speist. Der Lock-in-Verstärker 22 erhält über die Leitung 11 und eine Frequenzverdopplerschaltung 23 Taktsignale mit der doppelten Frequenz, mit der die Transmissionswellenlänge des Interferenzfilters 10 für die Derivativ-Spektrometrie gewobbelt wird.

Die Ausgangssignale der Lock-in-Verstärker 18, 19, 20 und 22 werden über in der Zeichnung nicht dargestellte Analog-Digital-Wandler digitalisiert und gelangen zu einem Meßwertspeicher 24.

Die dem Meßwertspeicher 24 vorgeschaltete Selektionselektronik 25 ist in Fig. 1 gestrichelt umrahmt. Die Selektionselektronik 25 liefert an den Meßwertspeicher 24 ein erstes Signal, das der effektiven Schichtdicke insbesondere des Blutes in der Probenhalterung 15 und damit der jeweiligen nichtkonstanten Ohrläppchendicke zugeordnet ist. Das zweite Signal liefert ein Maß für die jeweilige Intensität der Strahlungsquelle 1, so daß bei Änderungen der Intensität der Strahlungsquelle 1 aus diesem Signal Korrekturen für die übrigen Signale abgeleitet werden können, um zu verhindern, daß bei einer kleiner werdenden Intensität der Strahlungsquelle 1 eine erhöhte Absorption als Meßergebnis angezeigt

wird. Die absoluten Veränderungen der Stoffkonzentration bzw. des Glukosespiegels werden durch das dritte Ausgangssignal der Selektionselektronik 25 angezeigt, während das vierte mit Hilfe der Derivativ-Signale erzeugte Meßsignal ein Maß für die relativen Konzentrationsänderungen insbesondere der Glukoseschwankungen darstellt.

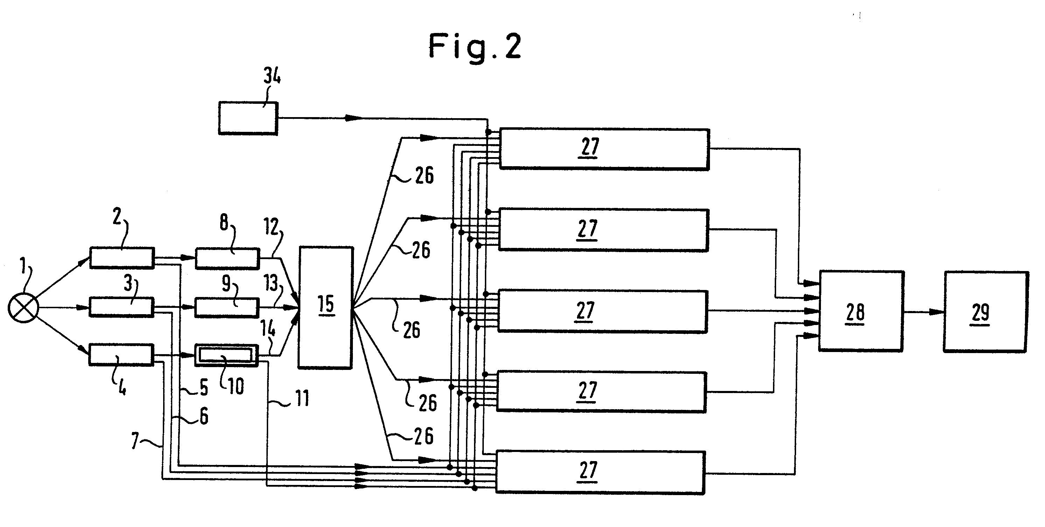

Für viele Anwendungen wären die ersten drei Signale ausreichend, nicht jedoch für die Bestimmung niedriger Konzentrationen oder die hochpräzise Glukosemessung im Blut des Ohrläppchens, weil die Absorption schwach ist und weil neben der Absorption auch die Brechung des Lichtes in der Meßprobe oder an Glukosemolekülen eine Rolle spielt. Wegen der auftretenden Brechung ist es zweckmäßig, die in Fig. 1 dargestellte und für die Messung bei einer Streuung ohne Brechung vorgesehene Vorrichtung in der in Fig. 2 dargestellten Weise zu ergänzen.

Bei dem in Fig. 2 dargestellten Ausführungsbeispiel einer Vorrichtung zur Messung der Absorption bei einer Streuung mit Brechung sind für entsprechende Bauteile die gleichen Bezugszeichen verwendet worden. Der Lichtweg von der Strahlungsquelle 1 über die drei Lichtkanäle bis zur Probenhalterung 15 entspricht dem in Fig. 1 dargestellten Ausführungsbeispiel. Das die Probenhalterung 15 verlassende Licht bestrahlt jedoch nicht einen einzelnen Detektor, sondern einen Ringdetektor, der das hinter der Probenhalterung 15 divergent austretende Lichtbündel erfaßt und es gestattet, die Lichtverteilung im Lichtbündel sowie Veränderungen der Lichtverteilung im Lichtbündel zu erfassen. Der Ringdetektor besteht beispielsweise aus einem zentralen scheibenförmigen lichtempfindlichen Element und mehreren, z.B. vier, konzentrisch um dieses angeordneten ringförmigen Detektorelementen. Eine solche Ringstruktur kann beispielsweise auch durch Diodenzeilen oder eine Diodenmatrix gebildet werden. Der Ringdetektor ist hinter der Probenhalterung 15 konzentrisch zum austretenden Meßstrahl angeordnet und liefert für sein zentrales Element sowie seine vier dieses umgebende Ringe Ausgangsspannungen, die über die Leitungen 26 zu Auswerteeinheiten 27 geführt werden. Jede der Auswerteeinheiten 27 entspricht der in Fig. 1 strichpunktiert umrahmten Auswerteeinheit 27. Auf diese Weise ist es durch die Vielzahl der Auswerteeinheiten 27 möglich, nicht nur Intensitätsveränderungen im Bereich der Transmissionswellenlängen der Interferenzfilter zu erfassen, sondern auch Veränderungen in der Intensitätsverteilung, die auf eine beispielsweise glukosebedingte Brechung zurückzuführen sind.

Die Signale der Auswerteeinheiten 27 gelangen zu einer Summierschaltung 28 und von dort zu einer Anzeigeeinheit 29. Bei dem in Fig. 1 dargestellten Ausführungsbeispiel ist der Ausgang der Auswerteeinheit 27 unmittelbar mit der Anzeigeeinheit 29 verbunden.

Neben dem Meßwertspeicher 24 enthält jede Auswerteeinheit 27 einen Kalibrierspeicher 30 mit einer Kalibriertabelle, eine Auswerteschaltung 31 und eine Kalibrierauswertungseinrichtung 32. Außerdem verfügt jede Auswerteeinheit 27 über einen Anfangswertspeicher 33. Die zum Kalibrieren benötigten Kalibrierwerte sind in den Fig. 1 und 2 durch das mit 34 bezeichnete Kästchen veranschaulicht.

Nachfolgend wird die Auswertung der Meßsignale für Glukose näher erörtert. Der aus dem Lichtwellenleiterende 14 austretende Meßstrahl ist im allgemeinen rotationssymmetrisch. Entsprechendes gilt für die auf den Detektor der Meßvorrichtung auftreffende Intensität I des Lichtes. Eine Konzentrationserhöhung von Glukose würde durch Absorption erwartungsgemäß eine gleichmäßige Intensitätsschwächung bewirken, während eine Konzentrationserniedrigung zunächst eine gleichmäßige Intensitätszunahme erwarten läßt. Überraschenderweise überlagert sich jedoch mit dem Absorptionseffekt ein Brechungseffekt, durch den das Licht ebenfalls konzentrationsabhängig umgelenkt wird. Dadurch kann sich die Absorption scheinbar erhöhen oder auch erniedrigen. Die durch die Überlagerung beider Effekte verursachten Intensitätsänderungen werden durch Auswerten der an den Leitungen 26 anstehenden Signale der verschiedenen konzentrisch angeordneten Ringelemente des Ringdetektors erfaßt.

Jede Auswerteeinheit 27 enthält daher den Anfangswertspeicher 33 für die Ausgangsintensitäten $I_i°$ und ermittelt für die einzelnen Ringelemente $i = 1...5$ die jeweiligen relativen Intensitätsverhältnisse $I_i/I_i°$, die ein proportionales Maß für die Konzentrationsänderung der Glukose sind.

Das Ziel der Verarbeitung der verschiedenen Signale ist die möglichst genaue Ermittlung der Intensitätsänderungen für jeden Zeitpunkt. Der Rechenablauf ist wie folgt: Für die Auswertung des Derivativ-Signals ist nach N. Hager und R. Stäudner (Technisches Messen 43 1976 S. 329 - 364) die Bestimmung des der Konzentration proportionalen Ausdrucks

$$C \propto \frac{1}{I} \frac{d^2 I}{d\lambda^2} \qquad (1)$$

erforderlich. Bezeichnet man die auf den i-ten Ring des Ringdetektors fallenden Signale für den ersten Lichtkanal (805 nm) mit $S1_i$, für den zweiten Lichtkanal (1300 nm) mit $S2_i$, für den dritten Lichtkanal mit $S3_i$ und für den Kanal des Derivativspektrometers mit $S3D_i$,

so gilt folgendes:

Während $d^2I/d\lambda^2$ dem Signal $S3D_i$ entspricht, kann anstelle von I $S3_i$ gesetzt werden, sofern die Geräteparameter und die Meßschichtdicke konstant bleiben. Nichtkonstante Verhältnisse können dadurch erfaßt werden, daß I mit dem Faktor $S1_i°/S1_i$ als Näherung für kleine Änderungen der Meßschichtdicke und dem Faktor $S2_i/S2_i°$ für Geräteparameter-Änderungen, speziell der Lampenintensität, zu multiplizieren ist. Dann berechnet sich die Konzentrationsänderung der Glukose $\Delta C_{iG}$ als jeweiliger Relativwert aus

$$\Delta C_{iG} \propto 1 - \frac{\dfrac{S3D_i}{S3D_i°}}{\dfrac{S3_i}{S3_i°}\dfrac{S1_i°}{S1_i}\dfrac{S2_i}{S2_i°}}, \qquad (2)$$

wobei der Index $°$ den gespeicherten Anfangswert darstellt. Diese Werte $\Delta C_{iG}$ können innerhalb einer Messung im Zentrum der Strahlung positiv und außen negativ oder umgekehrt sein. Deswegen ergibt sich die gesuchte Konzentrationsänderung $\Delta C_G$ aus

$$\Delta C_G = \sum_{i=1}^{5} |\Delta C_{iG}| \qquad (3)$$

Diese Werte sind Relativwerte und müssen über eine andere Methode absolut kalibriert werden.

Die Hardware für diese Rechen- und Kalibrieroperationen ist in Fig. 1 für eine Einelement-Detektor-Anordnung veranschaulicht, bei der keine räumliche Auflösung der Strahlung erfolgt, wenn der Brechungsindex die Absorptionsmessung unwesentlich beeinflußt. Für diesen Fall ist in Fig. 1 hinter dem Probenhalter 15 ein einzelnes Detektorelement 16 an Stelle des oben erwähnten Ringdetektors angebracht.

Bei dem Ausführungsbeispiel gemäß Fig. 2 werden die Anfangswerte $S1_1^o$, $S2_1^o$, $S3_1^o$ und $S3D_1^o$ aus der Selektionselektronik 25 abgerufen und in dem Anfangswertspeicher 33 abgelegt. Der Kalibrierspeicher 30 enthält die Kalibrierwerte, die extern durch den Einsatz einer anderen, bereits etablierten Methode 34 geliefert werden, und die dazugehörigen Meßwerte $S1_1^k$, $S2_1^k$, $S3_1^k$ und $S3D_1^k$. Beide Wertesätze mit den Indizes $^o$ und $^k$ werden dem Meßwertspeicher 24 entnommen, der die laufenden Meßwerte $S1_1$, $S2_1$, $S3_1$ und $S3D_1$ enthält. Aus den im Meßwertspeicher 24 und Anfangswertspeicher 33 enthaltenen Werten wird in der Auswerteschaltung 31 unter Einsatz der obigen Gleichung (2) mit $i = 1$ ein Relativwert für die Änderung der Glukosekonzentration ermittelt. Aus dem Vergleich mit den Kalibrierwerten aus dem Kalibrierspeicher 30 wird in der Kalibrierauswertungseinrichtung 32 die Glukosekonzentration bestimmt und mit Hilfe der Anzeigeeinheit 29 dargestellt. Die in Fig. 1 strichpunktiert umrahmte Auswerteinheit 27 wird bei dem in Fig. 1 dargestellten Ausführungsbeispiel für den einzig vorhandenen Detektor 16 und bei dem in Fig. 2 dargestellten Ausführungsbeispiel für jedes Ringelement verwendet.

Bei Meßproben wie Glukose im Blut, die neben der Absorption auch die Brechung des Lichts merklich beeinflussen, erfolgt zweckmäßigerweise eine räumliche Auflösung der detektierten Strahlung beispielsweise durch die Verwendung eines Ringdetektors mit fünf Elementen ($i = 1...5$). Für eine solche Ringdetektoranordnung erfolgt die Datenauswertung unter Einsatz mehrerer Auswerteeinheiten 27 gemäß Fig. 2. Der Eingangsteil mit der Strahlungsquelle 1, den Choppern 2, 3, 4 mit den Lichtschranken, den Interferenzfiltern 8, 9, 10 und der Probenhalterung 15 unterscheidet sich nicht von der Ein-Detektor-Anordnung gemäß Fig. 1. Anstelle eines Einzeldetektors werden jedoch bei dem Ausführungsbeispiel gemäß Fig. 2 fünf Detektor-Elemente eingesetzt, deren Signale jeweils getrennt, bzw. parallel mit Auswerteeinheiten 27 verarbeitet werden, deren Relativwerte in der Summierschaltung 28 nach Gleichung 3 aufsummiert und in der Anzeigeeinheit 29 dargestellt werden.

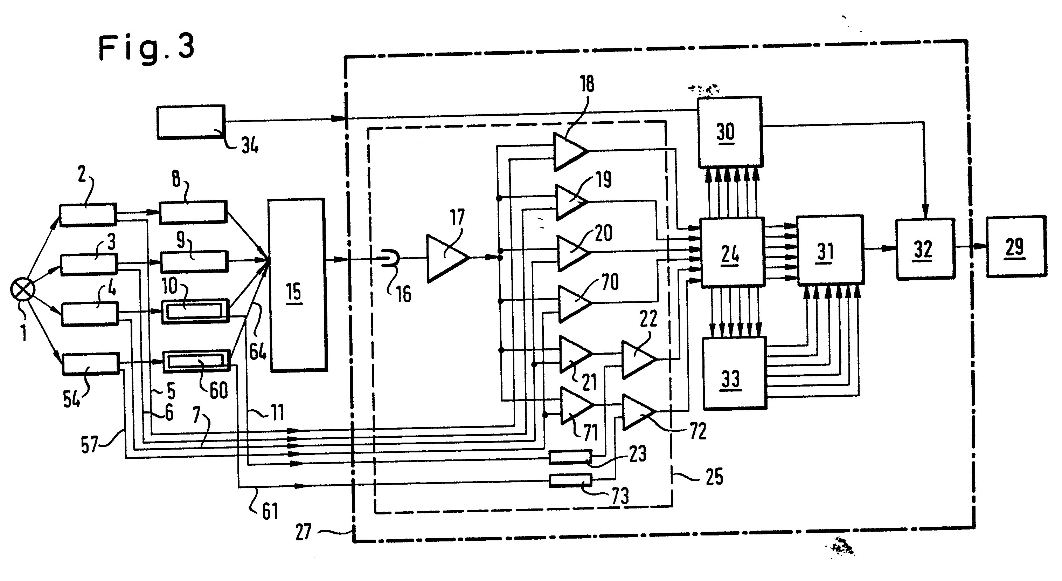

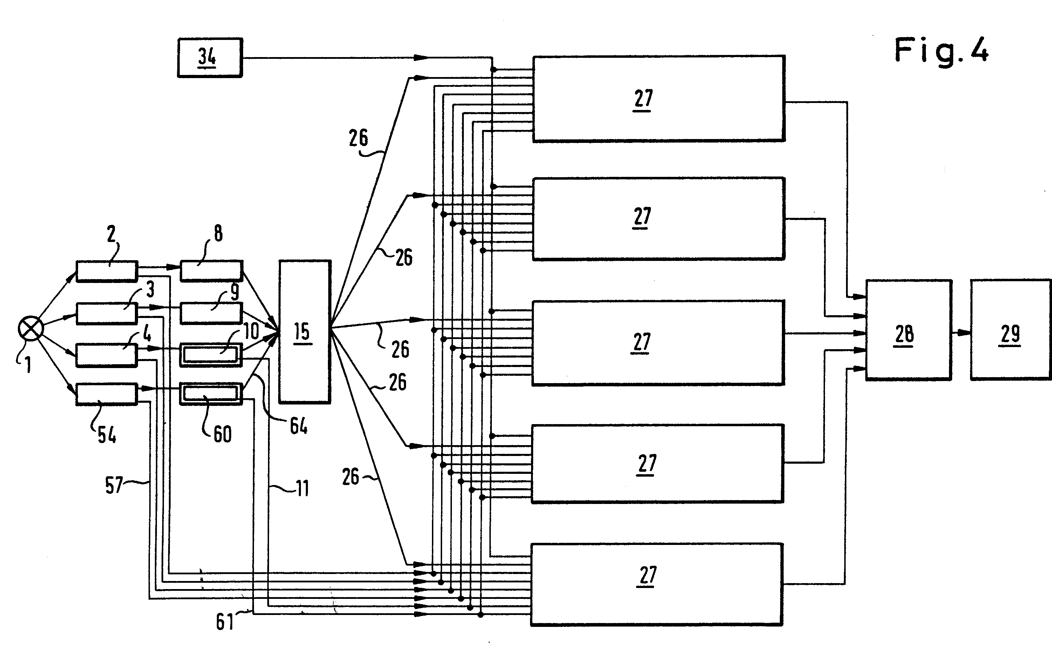

Während die in den Figuren 1 und 2 dargestellten Ausführungsbeispiele der Erfindung die Überwachung eines einzigen Stoffes zum Ziele haben, zeigen die Figuren 3 und 4 Ausführungsbeispiele der Erfindung, die die Überwachung zweier Stoffe in der Meßprobe gestatten. Aus der nachfolgenden Beschreibung geht für den Fachmann ohne weiteres auch hervor, daß die Vorrichtung zur kontinuierlichen Bestimmung von Konzentrationsänderungen in Stoffgemischen auch so erweitert werden kann, daß nicht nur zwei Stoffe, sondern eine Vielzahl von Stoffen in einem Stoffgemisch überwacht werden können, wenn für jeden Stoff ein Lichtkanal bereitgestellt wird, dessen Filter jeweils im Bereich der Absorptionsbande des zu überwachenden Stoffes liegt.

Bei den in den Figuren 3 und 4 dargestellten Ausführungsbeispielen sind die aus den Figuren 1 und 2 bereits bekannten Teile mit den gleichen Bezugszeichen versehen. Die Probenhalterung 15 kann für den Fall von Blutuntersuchungen als Ohrclip ausgebildet sein, das auf das Ohrläppchen eines Patienten aufgeklemmt ist. Dabei gestattet es beispielsweise die in den Figuren 3 und 4 dargestellte Vorrichtung, neben einer Überwachung der Glukose im Blut durch Abstimmen des dritten Interferenzfilters 10 auf eine Transmissionswellenlänge von 1600 nm zusätzlich das Blut des Patienten auf den Cholesteringehalt zu überprüfen, wenn ein weiteres Interferenzfilter 60 in der in Fig. 3 dargestellten Weise verwendet wird, um einen zusätzlichen Lichtkanal mit einer Transmissionswellenlänge von 1710 nm zwischen der Strahlungsquelle 1 und der Probenhalterung 15 bereitzustellen.

Der dem Cholesterin zugeordnete Lichtkanal empfängt das Licht der Strahlungsquelle 1 über einen vierten Chopper 54, dessen Chopperfrequenz sich von den Chopperfrequenzen der Chopper 2 bis 4 unterscheidet, so daß eine Separierung des Signals in der Auswerteeinheit 27 möglich ist.

Über eine Leitung 57 werden Steuersignale des Choppers 54 ausgekoppelt. Wie Fig. 3 entnommen werden kann, gelangen diese Steuersignale zu einem Lock-in-Verstärker 70 und einem weiteren Boxcar-Verstärker 71. Der Ausgang des Boxcar-Verstärkers 71 ist entsprechend dem Boxcar-Verstärker 21 mit einem Lock-in-Verstärker 72 verbunden, dem über einen Frequenzverdoppler 73 Taktsignale zugeführt werden. Aus diesem Grunde ist das vierte Interferenzfilter 60 elektrisch über eine Leitung 61 mit dem Eingang des Frequenzverdopplers 73 verbunden, so daß auch in dem zusätzlichen für die Cholesterinerfassung vorgesehenen Kanal eine Derivativ-Spektrometrie durchgeführt werden kann. In Fig. 3 erkennt man weiterhin einen Lichtwellenleiter 64, über den das Licht nach Durchgang durch das vierte Interferenzfilter 60 zur Probenhalterung 15 geleitet wird.

Während Fig. 3 eine Erweiterung um einen vierten Lichtkanal bzw. einen zweiten Meßkanal gegenüber Fig. 1 darstellt, zeigt Fig. 4 eine Meßbereichserweiterung für die in Fig. 2 dargestellte Anordnung und gestattet wie diese Anordnung eine Auswertung der räumlichen Lichtverteilung des den Probenhalter 15 verlassenden Lichtes. Auch in Fig. 4 sind Bauteile, die mit den Bauteilen in Fig. 2 übereinstimmen, mit den gleichen Bezugszeichen versehen. Zur Realisierung des zweiten Meßkanals bzw. des vierten Lichtkanals erkennt man, daß das Ausführungsbeispiel gemäß Fig. 4 neben dem vierten Chopper 54 und dem vierten Interferenzfilter 60 Auswerteeinheiten 27 aufweist, die statt sechs Eingängen wie in Fig. 2 jeweils acht Eingänge aufweisen. Die beiden zusätzlichen Eingänge der Auswerteeinheiten 27 entsprechen den Leitungen 61 und 57, die bereits im Zusammenhang mit Fig. 3 erörtert worden sind. Die Auswertung der verschiedenen Signale bei der in Fig. 4 dargestellten Vorrichtung erfolgt analog zur Auswertung bei der Vorrichtung gemäß Fig. 3, wobei jedoch zusätzlich der dem Cholesterin zugeordnete Kanal mit einer Transmissionswellenlänge von 1710 nm überwacht wird.

Wie bereits erwähnt, ist es möglich, die oben beschriebenen Anordnungen um weitere Kanäle zu erweitern oder durch die Wahl der verschiedenen Transmissionsfrequenzen zur Überwachung anderer Stoffe als Glukose und Cholesterin im Blut zu verwenden. Die jeweiligen Transmissionswellenlängen für die Interferenzfilter 8 bis 10 und 60 lassen sich leicht aus den Absorptionsbanden der in den Stoffgemischen vorhandenen Stoffe direkt bestimmen oder experimentell ermitteln, wenn das Transmissionsverhalten der Stoffgemische noch unbekannt ist.

PATENTANSPRÜCHE

1. Vorrichtung zur kontinuierlichen Bestimmung von Konzentrationsänderungen in Stoffgemischen, mit einer Strahlungsquelle, deren Licht die Meßprobe durchstrahlt und von einer Detektoranordnung zur Bestimmung der stoffspezifischen Absorptionsmessung erfaßt wird, dadurch gekennzeichnet, daß die Strahlungsquelle (1) eine breitbandige Lampe ist, deren Licht über eine Optik in mehrere Lichtkanäle mit jeweils einem eine individuelle Frequenz aufweisenden Chopper (2, 3, 4, 54), einem eine vorbestimmte Transmissionswellenlänge auszeichnenden Filter (8, 9, 10, 60) und einem Lichtwellenleiter (12, 13, 14, 64) in die Meßprobe (15) eingekoppelt ist, daß das Ausgangssignal der Detektoranordnung (16) über den Lichtkanälen zugeordnete auf die Chopperfrequenzen abgestimmte Lock-in-Verstärker (18, 19, 20, 22, 70, 72) an eine Auswertevorrichtung (27,28, 29, 31) angeschlossen ist und daß die Transmissionswellenlänge des ersten Filters (8) in einem hauptsächlich auf die Probenschichtdicke ansprechenden Bereich, die des zweiten Filters (9) in einem Bereich, wo die Meßprobe nur schwach absorbiert, und die der übrigen Filter (10, 60) im Bereich der Absorptionsbanden der Stoffe liegt, deren Konzentrationsänderungen bestimmt werden sollen.

2. Vorrichtung nach Anspruch 1, dadurch gekennzeichnet, daß die Detektoranordnung wenigstens einen von der Strahlung aller Lichtkanäle beleuchteten Detektor (16) aufweist, dessen Ausgangssignal den einzelnen Lichtkanälen zugeordnete mit den Choppern (2, 3, 4, 54) synchronisierte Lock-in-Verstärker (18, 19, 20, 22, 70, 72) speist, deren den einzelnen Lichtkanälen zugeordnete Ausgangssignale eine Auswerteschaltung (31) beaufschlagen.

3. Vorrichtung nach Anspruch 2, d a d u r c h   g e - k e n n z e i c h n e t ,   daß die Detektoranordnung eine Vielzahl von Detektorelementen aufweist, die rotationssymmetrisch zur optischen Achse des die Meßprobe (15) verlassenden Lichtbündels als scheibenförmiges Zentralelement mit konzentrischen Ringelementen geschaltet sind und daß dem Zentralelement sowie jedem Ringelement (26) eine Auswerteeinheit (27) mit für jeden Lichtkanal ausgelegten Lock-in-Verstärkern (18, 19, 20, 22, 70, 72) zugeordnet sind (Figuren 2 und 4).

4. Vorrichtung nach Anspruch 3, d a d u r c h   g e - k e n n z e i c h n e t ,   daß die die Ergebnisse für die einzelnen Ringelemente (26) erfassenden Auswerteschaltungen (27) an eine Summierschaltung (28) angeschlossen sind, deren Ausgang eine Anzeigeeinrichtung (29) speist.

5. Vorrichtung nach einem der vorstehenden Ansprüche, d a d u r c h   g e k e n n z e i c h n e t ,   daß die Strahlungsquelle (1) eine Halogenlampe ist.

6. Vorrichtung nach einem der vorstehenden Ansprüche, d a d u r c h   g e k e n n z e i c h n e t ,   daß die Lichtwellenleiter (12, 13, 14) mit einem Ohrclip zum Aufstecken auf das Ohrläppchen eines Patienten gekoppelt sind, dessen Ausgangssignal die Detektoranordnung (16) speist und daß zur Überwachung der Glukosekonzentration im Blut die Transmissionswellenlänge der Filter (8, 9, 10) 805 nm für den ersten, 1300 nm für den zweiten und 1600 nm für einen dritten Lichtkanal betragen.

7. Vorrichtung nach einem der vorstehenden Ansprüche, dadurch gekennzeichnet, daß der dritte Lichtkanal einem ersten zu überwachenden Stoff und ein vierter Lichtkanal einem zweiten zu überwachenden Stoff zugeordnet ist.

8. Vorrichtung nach einem der vorstehenden Ansprüche, dadurch gekennzeichnet, daß die Filter (8, 9, 10, 60) Interferenzfilter sind.

9. Vorrichtung nach einem der vorstehenden Ansprüche, dadurch gekennzeichnet, daß das bzw. die der Absorptionsbande der zu erfassenden Stoffe zugeordneten Filter (10, 60) periodisch verstimmbar sind, wobei die zugeordneten Lichtkanäle eine Anordnung für die Derivativ-Spektrometrie (11, 21, 22, 23, 61, 71, 72, 73) darstellen.

10. Vorrichtung nach einem der Ansprüche 2 bis 8, dadurch gekennzeichnet, daß die Auswerteeinheiten (27) einen Kalibrierspeicher (30) aufweisen.

11. Vorrichtung nach einem der vorstehenden Ansprüche, dadurch gekennzeichnet, daß die Chopper (2, 3, 4, 54) durch Segmente auf einem gemeinsamen Chopperrad ausgebildet sind.

0226822

Fig.1

0226822

Fig.2

# Fig.3

HELLIGE GMBH - PW-P505-EP

0226822

0226822

Fig.4